# EUROPÄISCHE PATENTANMELDUNG

(11) **EP 1 933 133 A2**
(43) Veröffentlichungstag der Anmeldung: **18.06.2008**
(21) Anmeldenummer: 07015562.7
(22) Anmeldetag: 08.08.2007
(51) Int. Cl.: G01N 27/04

(54) **Anordnung zur Messung des Restfeuchtegehaltes von bahnartigen Materialien**

(30) Priorität: 14.12.2006 DE 202006019008 U
(71) Anmelder: Mahlo GmbH & Co. KG, 93342 Saal/Donau (DE)
(72) Erfinder: Schmidl, Karl-Heinz, 93309 Kelheim (DE)
(74) Vertreter: Glück, Martin

(57) **Zusammenfassung**

Die Erfindung betrifft eine Anordnung und ein zugehöriges Verfahren zur Messung des Restfeuchtegehaltes (RF) eines bahnartigen Materials (4) bestehend aus zumindest einer Messkopfeinheit (6) mit einer ersten Sensoreinheit (8) und einer zweiten gegenüberliegenden Sensoreinheit (8), wobei die in einer Förderrichtung (FR) bewegte Materialbahn (4) zwischen der ersten und zweiten Sensoreinheit (8, 3) geführt ist und mit der ersten und zweiten Sensoreinheit (8, 3) in Kontakt steht. Besonders vorteilhaft ist die erste Sensoreinheit (8) durch eine elektrisch leitfähige Messkugel gebildet, die drehbar in der Messkopfeinheit (6) gelagert ist.

## Beschreibung

Die Erfindung betrifft eine Anordnung und ein Verfahren zur Messung der Restfeuchtegehaltes von bahnartigen Materialien nach dem Oberbegriff des Patentanspruches 1 und 12.

Anordnungen zur Bestimmung des Restfeuchtegehaltes bzw. der Restfeuchtigkeit von bahnförmigen Materialien bzw. Materialbahnen wie beispielsweise Textilien, Papier oder speziellen Kunststofffolien sind hinlänglich bekannt.

Die Bestimmung des Restfeuchtegehaltes von flächigen Materialien, insbesondere von Textilien, Papier und speziellen Kunststofffolien unter Nutzung deren Leitfähigkeit - auch als konduktometrische Messung bezeichnet- ist hinlänglich bekannt. Hierzu wird der logarithmische Zusammenhang des elektrischen Widerstandes des bahnartigen Materials und der Restfeuchtigkeit ausgewertet. Bei derartigen flächigen Gebilden ist dieser Zusammenhang weitgehend unabhängig von der Flächenmasse des bahnartigen Materials.

Zur Führung der flächigen Materialbahn weisen bekannte Feuchtemessanordnungen üblicherweise Walzen- oder Rolleneinheiten auf, welche ortsfest und quer zur Förderrichtung der Bahn des flächigen Materials angeordnet sind. Zur Messung des elektrischen Widerstandes des zwischen zwei Walzen- oder Rolleneinheiten geführten flächigen bahnförmigen Materials werden zumindest zwei Messelektroden vorgesehen, welche beispielsweise an der Oberfläche der Walzen- oder Rolleneinheiten angeordnet sind und somit das zu vermessende bahnförmige Material berühren.

Aus der DE 100 25 902 A1 ist beispielsweise eine Feuchtemessanordnung zur Bestimmung des Restfeuchtegehaltes von Textilien, Papier oder speziellen Kunststofffolien bekannt, bei der eine von der zu vermessenden Materialbahn angetriebene Messwalze vorgesehen ist. Die Messwalze weist eine segmentierte Oberfläche auf, wobei die Oberflächensegmente jeweils durch leitfähige Umfangsbereiche gebildet sind, die über hochohmige Isolationsabschnitte voneinander getrennt sind. Durch den berührenden Kontakt der voneinander getrennten leitfähigen Umfangsbereiche mit dem flächigen bahnförmigen Material bildet sich ein Strompfad aus, und zwar dann wenn der Isolationswiderstand der Isolationsabschnitte wesentlich größer als derjenige des flächigen Materials selbst ist. Die entlang des Strompfades fließende Stromstärke wird gemessen und abhängig davon die Leitfähigkeit des flächigen bahnförmigen Materials an der jeweils lokalen Messstelle ermittelt. Hierbei ist die Messstelle durch die Anordnung des Isolationsabschnittes bzgl. der Materialbahn vorgegeben. Von der ermittelten Leitfähigkeit kann in an sich bekannter Weise auf die Restfeuchtigkeit des flächigen bahnförmigen Materials geschlossen werden. Nachteilig ermöglicht die beschriebene Anordnung keine exakte Bestimmung der Verteilung des Restfeuchtegehaltes quer zum flächigen bahnförmigen Material, d.h. senkrecht zur Förderrichtung. Einzelne Verarbeitungsprozesse, beispielsweise ein gezieltes Befeuchten derartiger flächiger bahnförmiger Materialien erfordern jedoch eine exakte Bestimmung der Restfeuchtigkeitsverteilung quer zur Bahn.

Bekannte Anordnungen zur Ermittlung der Verteilung der Restfeuchtigkeit quer zur Bahn basieren auf berührungslosen Messverfahren, bei denen zumindest ein Infrarot- oder Mikrowellensensor quer zu Bahn, d.h. traversierend geführt wird. Nachteilig sind derartige Messanordnungen prinzipbedingt sehr kostenintensiv und äußerst empfindlich im Hinblick auf die herrschender Umgebungsbedingungen wie beispielsweise Hitze, Dampf, etc.

Die Aufgabe der vorliegenden Erfindung ist darin zu sehen, eine Anordnung und ein Verfahren zur Bestimmung des Restfeuchtegehaltes eines flächigen bahnförmigen Materials anzugeben, mittels der bzw. dem technisch einfach, zuverlässig und kostengünstig der Restfeuchtegehalt sowohl lokal als auch dessen Verteilung quer zur Materialbahn bestimmt werden kann.

Die Aufgabe wird ausgehend vom Oberbegriff des Patentanspruches 1 und 12 durch jeweils deren kennzeichnende Merkmale gelöst. Vorteilhafte Weiterbildungen der neuerungsgemäßen Anordnung sind den weiteren abhängigen Ansprüchen zu entnehmen.

Ein wesentlicher Gedanke der Erfindung ist darin zu sehen, dass die erste Sensoreinheit durch eine elektrisch leitfähige Messkugel gebildet ist, die drehbar in der Messkopfeinheit gelagert ist. Mittels der Ausbildung der ersten Sensoreinheit als elektrisch leitfähige Messkugel kann besonders vorteilhaft eine kontinuierliche Messung der Feuchtigkeitsverteilung sowohl lokal als auch quer zur Materialbahn durchgeführt werden. Hierbei werden durch die drehbare Lagerung der Messkugel in der Messkopfeinheit die aufgrund des Kontaktes zur Materialbahn auf die elektrisch leitfähige Messkugel wirkenden Bewegungskräfte sowohl in Förderrichtung als auch quer zur Förderrichtung aufgenommen und dadurch ein zuverlässiger Kontakt zur Materialbahn sichergestellt.

Weiterhin vorteilhaft ist die zweite Sensoreinheit als Gegenwalzeneinheit realisiert und die Messkopfeinheit traversierend ausgebildet. Somit kann mittels der neuerungsgemäßen Anordnung die Verteilung des Restfeuchtegehaltes quer zur Materialbahn exakt ermittelt werden. Die Messkopfeinheit ist hierzu an einer Traversiervorrichtung angeordnet, die ortsfest und quer zur bewegten Materialbahn vorgesehen ist, wobei die Traversiervorrichtung parallel zur Gegenwalzeneinheit verläuft.

Ferner ist der Abstand zwischen der elektrisch leitfähigen Messkugel und der zweiten Sensoreinheit, insbesondere der Gegenwalzeneinheit einstellbar und die elektrisch leitfähige Messkugel entlang einer nahezu senkrecht zur Materialbahn verlaufenden Bewegungsachse in der Messkopfeinheit beweglich gelagert. Vorteilhaft können somit Materialbahnen unterschiedlicher Dicke vermessen werden und ggf. durch die Verarbeitung der Materialbahn bedingte Abweichungen der Dicke, beispielsweise aufgrund von in einer Textilbahn vorgesehenen Nähten ausgeglichen werden.

Die elektrisch leitfähige Messkugel wirkt mit einem vorgebbaren Anpressdruck auf die Materialbahn ein, der einstellbar ist und beispielsweise über eine Federeinheit oder ein pneumatisches Element erzeugt wird. Die Höhe des Anpressdruckes ist vorteilhaft derart gewählt, dass ein ausreichend guter Kontakt der Messkugel bzw. der Gegenwalzeneinheit zur Materialbahn vorliegt, jedoch keine bleibenden Abdrücke auf der Warenbahn entstehen.

Die Messkopfeinheit weist zur Aufnahme der elektrisch leitfähigen Messkugel ein Gehäusemodul auf, dass eine vorzugsweise kreisförmige Öffnung umfasst, aus der die im Gehäusemodul aufgenommene elektrisch leitfähige Messkugel zumindest teilweise in Richtung der Gegenwalzeneinheit herausragt. Besonders vorteilhaft ist zwischen dem Gehäusemodul und der Messkugel und der Öffnung und der Messkugel ein Luftspalt vorgesehen ist, der mit Druckluft beaufschlagt wird. Die Öffnung kann in einer bevorzugten Ausführungsform mit einem kreisförmigen Dichtungslippenelement versehen sein, welches den Spalt zumindest teilweise abdeckt. Vorteilhaft wird durch das sich im Luftspalt ausbildende Luftkissen und das Dichtungslippenelement das Eindringen von Schmutz, beispielsweise Staub, Feuchtigkeit oder Flusen, etc. in den Lagersitz der Messkugel vermieden.

Darüber hinaus können zur Herstellung einer leitfähigen Verbindung zur Messkugel in der Messkopfeinheit zumindest ein Schleifkontaktelement oder zumindest ein leitfähiges Lagerelement vorgesehen sein.

Im Folgenden wird die Erfindung anhand mehrerer Zeichnungen an einem Ausführungsbeispiel näher beschrieben. Es zeigen:
- Fig. 1: beispielhaft einen schematischen Querschnitt entlang der Y-Z-Ebene durch die neuerungsgemäße Feuchtigkeitsmessanordnung,
- Fig. 2: beispielhaft einen schematischen Querschnitt einen Querschnitt entlang der X-Z-Ebene durch die neuerungsgemäße Feuchtigkeitsmessanordnung,
- Fig. 3: beispielhaft einen schematischen Querschnitt entlang der Y-Z-Ebene durch die Messkopfeinheit der neuerungsgemäßen Feuchtigkeitsmessanordnung und
- Fig. 4: beispielhaft in einem Diagramm die über die neuerungsgemäße Feuchtigkeitsmessanordnung ermittelte Feuchtigkeitsverteilung.

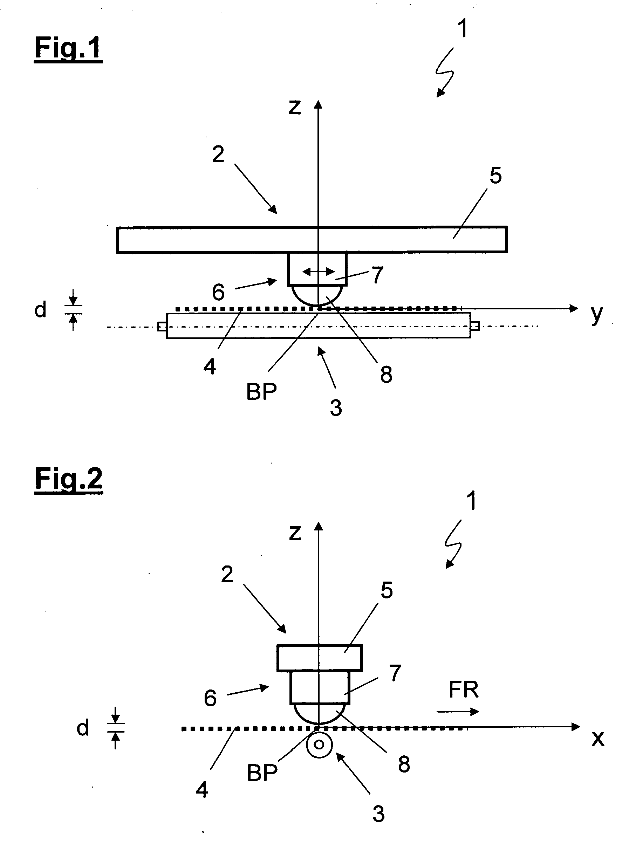

In den Figuren 1 und 2 ist beispielhaft jeweils in einem schematischen Blockschaltbild ein Querschnitt durch die erfindungsgemäße Feuchtigkeitsmessanordnung 1 dargestellt, und zwar entlang der Y-Z-Ebene bzw. der X-Z-Ebene des in den Figuren dargestellten Koord i natensystems.

Die Feuchtigkeitsmessanordnung 1 besteht beispielhaft aus einer Messeinrichtung 2 und einer Gegenwalzeneinheit 3, wobei zwischen der Messeinrichtung 2 und der Gegenwalzeneinheit 3 das Messobjekt, und zwar das zu vermessende flächige bahnförmige Material bzw. die Materialbahn 4 geführt ist. Die Gegenwalzeneinheit 3 bildet hierbei eine Sensoreinheit aus und ist beispielsweise ortsfest und quer zur Materialbahn 4 angeordnet. Das flächige bahnförmige Material 4 kann beispielsweise eine Textil- oder Papierbahn oder eine Bahn einer speziellen Kunststofffolie sein.

Die jeweilige Materialbahn 4 wird in an sich bekannter Weise mittels in den Figuren nicht dargestellter walzen- oder rollenförmigen Antriebseinheiten in eine Förderrichtung FR bewegt. Die Förderrichtung FR stimmt im vorliegenden Ausführungsbeispiel mit der X-Achse des in den Figuren dargestellten Koordinatensystems überein.

Die Messeinrichtung 2 weist eine Traversiervorrichtung 5 auf, an der eine Messkopfeinheit 6 beweglich vorgesehen ist. Die Traversiervorrichtung 5 ist vorzugsweise ortsfest, quer zur Materialbahn 4 und parallel zur Gegenrolleneinheit 3 angeordnet. Hierbei ist die Messkopfeinheit 6 derart beweglich an der Traversiervorrichtung 5 vorgesehen, dass diese zur Messung der Verteilung der Restfeuchtegehaltes quer zur Bahn 4 traversierbar ist, d.h. die Messkopfeinheit 6 ist entlang der Y-Achse gesteuert über die Traversiervorrichtung 5 verschiebbar.

Die Messkopfeinheit 6 weist ferner ein Gehäusemodul 7 auf, in dem eine als elektrisch leitfähige Messkugel ausgebildete weitere Sensoreinheit 8 zumindest teilweise aufgenommen ist. Die elektrisch leitfähige Messkugel 8 ist beispielsweise im Gehäusemodul 7 drehbar gelagert und steht im Betriebszustand mit der Materialbahn 4 in Kontakt. Aufgrund der Ausbildung der weiteren Sensoreinheit als elektrisch leitfähige Messkugel 8 können die von der Materialbahn 4 auf die traversierende Messkopfeinheit 6 übertragenen Bewegungskomponenten in der X-Y-Ebene aufgenommen werden, wodurch ein stets zuverlässiger Kontakt zwischen der Materialbahn 4 und der Messkugel 8 gewährleistet ist. Zusätzlich ist beispielsweise eine Lagerung der elektrisch leitfähigen Messkugel 8 im Gehäusemodul 7 entlang der Z-Achse, d.h. nahezu senkrecht zur Materialbahn 4 vorgesehen, über welche unterschiedlich dicke Abschnitte der zu vermessenden Materialbahn 4 ausgeglichen werden können.

Die Messkugel 8 wirkt mit der Gegenwalzeneinheit 3 zusammen, wobei diese einen Abstand d zueinander aufweisen, der in etwa gleich oder kleiner als die Dicke der zu vermessenden Materialbahn 4 ist. Der Abstand d zwischen der elektrisch leitfähigen Messkugel 8 und der Oberfläche der Gegenwalzeneinheit 3 ist ebenfalls einstellbar, so dass flächige bahnförmige Materialien 4 unterschiedlicher Dicke und Oberflächeneigenschaften mittels der Feuchtigkeitsmessanordnung 1 vermessen werden können. Die elektrisch leitfähige Messkugel 8 kann beispielsweise abhängig von der jeweils zu vermessenden Materialbahn 4 einen Radius R zwischen 20 und 200 mm aufweisen.

Der Restfeuchtegehalt des bahnartigen Materials 4 wird beispielsweise gemäß einem an sich bekannten konduktometrischen Messverfahren bestimmt, und zwar wird zwischen der elektrisch leitfähigen Messkugel 8 und der Gegenwalzeneinheit 3 eine elektrische Spannung angelegt, so dass sich im jeweiligen Berührungspunkt BP zwischen der elektrisch leitfähigen Messkugel 8 und der Materialbahn 4 bzw. der Gegenwalzeneinheit 3 und der Materialbahn 4 ein elektrischer Stromflusspfad ausbildet. Die Stromstärke des über den Stromflusspfad fließenden elektrischen Stromes wird über die Feuchtemessanordnung 1 bestimmt und abhängig davon der elektrische Widerstand der Materialbahn 4 am Berührungspunkt BP ermittelt.

Im Anschluss daran wird unter Ausnutzung des logarithmischen Zusammenhanges zwischen dem gemessenen elektrischen Widerstand und der Restfeuchtigkeit des flächigen bahnartigen Materials 4 der Restfeuchtegehalt am Berührungspunkt BP ermittelt. Der Berührungs- bzw. Messpunkt BP wandert hierbei aufgrund der traversierenden Anordnung der Messkopfeinheit 6 an der Traversiervorrichtung 5 jeweils quer zur Materialbahn 4 und parallel zur Gegenwalzeneinheit 3. Dies ermöglicht eine Messung der Verteilung des Restfeuchtegehaltes der bewegten Materialbahn 4.

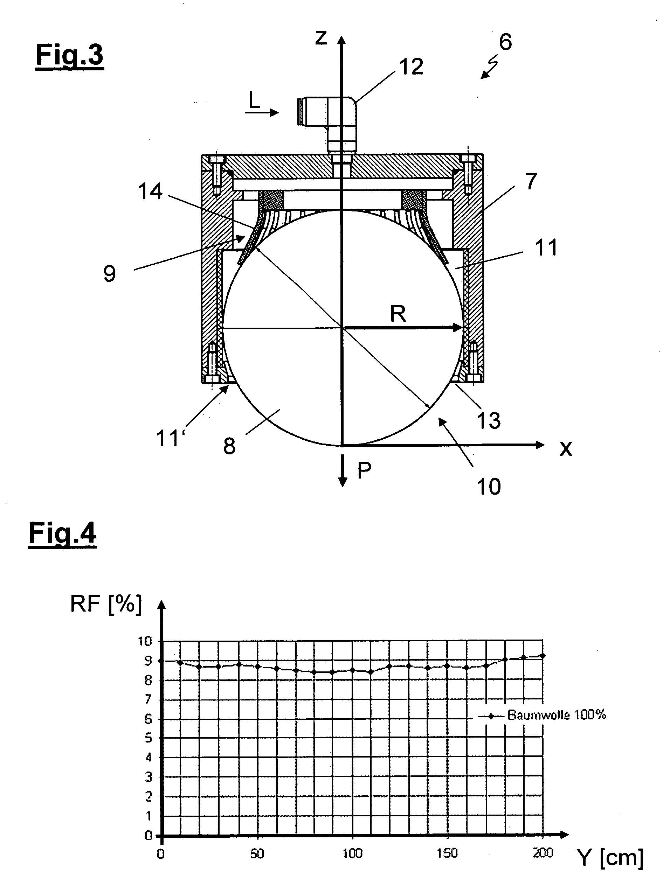

In Figur 3 ist beispielhaft der Aufbau einer Messkopfeinheit 6 in einer schematischen Schnittdarstellung entlang der X-Z-Ebene dargestellt. Das beispielsweise mehrteilig ausgebildete Gehäusemodul 7 weist einen Innenraum 9 zur zumindest teilweisen Aufnahme der elektrisch leitfähigen Messkugel 8 auf. Vorzugsweise ist an der zur Gegenwalzeneinheit 3 gerichteten Seite des Gehäusemoduls 7 zumindest eine beispielsweise kreisförmige Öffnung 10 vorgesehen, aus der die im Innenraum 9 des Gehäusemoduls 7 befindliche elektrisch leitfähige Messkugel 8 zumindest teilweise entlang der Z-Achse und in Richtung der Gegenwalzeneinheit 3 herausragt. Der Durchmesser der Öffnung 10 ist hierbei kleiner als der Durchmesser der elektrisch leitfähigen Messkugel 8 gewählt.

Zwischen dem Gehäusemodul 7 und der Messkugel 8 bzw. der Öffnung 10 und der Messkugel 8 ist vorzugsweise ein Luftspalt 11, 11' vorgesehen, der beispielsweise über eine Zuführungsleitung 12 mit Druckluft L beaufschlagt wird. Durch die Zuführung von Druckluft L bildet sich im Innenraum 9 des Gehäusemoduls 7 ein Luftkissen aus, welches die elektrisch leitfähige Messkugel 8 umgibt. Die über die Zuführungsleitung 12 zugeführte Druckluft L entweicht über den Luftspalt 11' der Öffnung 10 aus dem Innenraum 9 des Gehäusemoduls 7 und verhindert somit ein Eindringen von Schmutzpartikeln in Form von Staub, Feuchtigkeit oder Flusen etc. in den Innenraum 9 des Gehäusemoduls 7. Hierdurch wird verhindert, dass eine verschmutzungsbedingte Beeinträchtigung der Funktionsweise der Messkugel 8 entsteht. In einer bevorzugten Ausführungsform kann die Öffnung 10 mit einem kreisförmigen Dichtungslippenelement 13 versehen sein, welches den Spalt 11' zumindest teilweise abdeckt und somit ebenfalls ein Eindringen von Schmutzpartikeln in den Innenraum 9 des Gehäusemoduls 7 verhindert.

Zur elektrisch leitfähigen Verbindung der Messkugel 8 mit der Messkopfeinheit 6 bzw. einer im Innenraum 9 des Gehäusemoduls 7 vorgesehenen Messelektrode (nicht in den Figuren dargestellt) ist vorzugsweise an dem vom Berührungspunkt BP gegenüberliegenden Randabschnitt der Messkugel 8 zumindest ein Schleifkontaktelement 14 vorgesehen, welches mit der Messkugel 8 in Kontakt steht. Alternativ oder zusätzlich zum Vorsehen von derartigen Schleifkontaktelementen 14 kann die Lagerung der elektrisch leitfähigen Messkugel 8 durch zumindest ein leitfähiges Lagerelement gebildet sein.

Die elektrisch leitfähige Messkugel 8 wirkt in einer bevorzugten Ausführungsform mit einem vorgegebenen Anpressdruck P auf die Materialbahn 4 und somit auf die Gegenwalzeneinheit 3 ein. Hierbei ist der in Richtung der Z-Achse wirkende Anpressdruck P über die Messkopfeinheit 6 einstellbar. Beispielsweise kann hierfür eine in den Figuren nicht dargestellte Federeinheit oder ein pneumatisches Element verwendet werden. Der Anpressdruck P wird hierbei derart gewählt, dass ein ausreichend guter Kontakt der elektrisch leitfähigen Messkugel 6 und der Gegenwalzeneinheit 3 zur Materialbahn 4 vorliegt, jedoch keine bleibenden Abdrücke auf der Materialbahn 4 oder eine Beschädigung dieser entstehen.

In Figur 4 ist beispielhaft in einem Diagramm eine mittels der Feuchtigkeitsmessanordnung 1 ermittelte Verteilung des Restfeuchtegehaltes RF quer zur Materialbahn dargestellt. Beispielhaft wurde als Messobjekt eine aus 100 % Baumwolle bestehende Textilbahn gewählt, welche der Feuchtigkeitsmessanordnung 1 zugeführt wird.

Entlang der Abszisse des Diagramms ist die jeweilige Y-Koordinate in cm (Breite der zu vermessenen Textilbahn) und entlang der Ordinate der an den unterschiedlichen Berührungs- bzw. Messpunkten BP gemessene Restfeuchtegehalt RF in Prozent aufgetragen. Gemäß Figur 4 weisen die einzelnen Messpunkte BP einen Abstand von beispielsweise 10 cm auf. Der gemessene Restfeuchtegehalt RF quer zur Textilbahn 4 beträgt hierbei beispielsweise zwischen ca. 8 und 9 Prozent.

Die Erfindung wurde voranstehend an einem Ausführungsbeispiel beschrieben. Es versteht sich von selbst, dass zahlreiche Änderungen sowie Modifikationen möglich sind, ohne dass dadurch der der Erfindung zugrunde liegende Gedanke verlassen wird.

### Bezugszeichenliste

- 1: Feuchtigkeitsmessanordnung
- 2: Messeinrichtung
- 3: zweite Sensoreinheit bzw. Gegenwalzeneinheit
- 4: Materialbahn bzw. flächiges bahnartiges Material
- 5: Traversiervorrichtung
- 6: Messkopfeinheit
- 7: Gehäusemodul
- 8: erste Sensoreinheit bzw. elektrisch leitfähige Messkugel
- 9: Innenraum
- 10: Öffnung
- 11: Luftspalt
- 11': Luftspalt
- 12: Zuführungsleitung
- 13: Dichtungslappenelement
- 14: Schleifkontaktelemente

- BP: Berührungs- bzw. Messpunkt
- d: Abstand
- L: Druckluft
- FR: Förderrichtung
- P: Anpressdruck
- R: Radius
- RF: Restfeuchtegehalt
- X, Y, Z: Achsen eines Koordinatensystems

## Patentansprüche

1. Anordnung zur Messung des Restfeuchtegehaltes (RF) eines bahnartigen Materials (4) bestehend aus zumindest einer Messkopfeinheit (6) mit einer ersten Sensoreinheit (8) und einer zweiten gegenüberliegenden Sensoreinheit (8), wobei die in einer Förderrichtung (FR) bewegte Materialbahn (4) zwischen der ersten und zweiten Sensoreinheit (8, 3) geführt ist und mit der ersten und zweiten Sensoreinheit (8, 3) in Kontakt steht, **dadurch gekennzeichnet, dass** die erste Sensoreinheit (8) durch eine elektrisch leitfähige Messkugel gebildet ist, die drehbar in der Messkopfeinheit (6) gelagert ist.

2. Anordnung nach Anspruch 1, **dadurch gekennzeichnet, dass** die zweite Sensoreinheit durch eine Gegenwalzeneinheit (3) gebildet ist und/oder dass die Messkopfeinheit (6) traversierend ausgebildet ist.

3. Anordnung nach Anspruch 2, **dadurch gekennzeichnet, dass** die Messkopfeinheit (6) an einer Traversiervorrichtung (5) angeordnet ist, die ortsfest und quer zur bewegten Materialbahn (4) vorgesehen ist.

4. Anordnung nach Anspruch 3, **dadurch gekennzeichnet, dass** die Traversiervorrichtung (5) parallel zur Gegenwalzeneinheit (3) angeordnet ist.

5. Anordnung nach einem der Ansprüche 1 bis 4, **dadurch gekennzeichnet, dass** der Abstand (d) zwischen der elektrisch leitfähigen Messkugel (8) und der zweiten Sensoreinheit (3), insbesondere der Gegenwalzeneinheit einstellbar ist und/oder dass die Messkopfeinheit (6) zur Aufnahme der elektrisch leitfähigen Messkugel (8) ein Gehäusemodul (7) aufweist.

6. Anordnung nach einem der Ansprüche 1 bis 5, **dadurch gekennzeichnet, dass** die elektrisch leitfähige Messkugel (8) entlang einer nahezu senkrecht zur Materialbahn (4) verlaufenden Achse (Z) in der Messkopfeinheit (6) beweglich gelagert ist und/oder dass die elektrisch leitfähige Messkugel (8) mit einem vorgebbaren Anpressdruck (P) auf die Materialbahn (4) einwirkt.

7. Anordnung nach Anspruch 6, **dadurch gekennzeichnet, dass** der Anpressdruck (P) einstellbar ist und/oder dass zur Erzeugung des Anpressdruckes (P) eine Federeinheit oder ein pneumatisches Element vorgesehen sind.

8. Anordnung nach einem der Ansprüche 5 bis 7, **dadurch gekennzeichnet, dass** das Gehäusemodul (7) eine vorzugsweise kreisförmige Öffnung (10) aufweist, aus der die im Gehäusemodul (7) aufgenommene elektrisch leitfähige Messkugel (8) zumindest teilweise herausragt.

9. Anordnung nach Anspruch 8, **dadurch gekennzeichnet, dass** zwischen dem Gehäusemodul (7) und der Messkugel (8) und der Öffnung (10) und der Messkugel (8) ein Luftspalt (11, 11') vorgesehen ist, der mit Druckluft (L) beaufschlagt wird.

10. Anordnung nach Anspruch 9, **dadurch gekennzeichnet, dass** die Öffnung (10) mit einem kreisförmigen Dichtungslippenelement (13) versehen ist, welches den Luftspalt (11') zumindest teilweise abdeckt.

11. Anordnung nach einem der Ansprüche 1 bis 14, **dadurch gekennzeichnet, dass** zur Herstellung einer leitfähigen Verbindung zur Messkugel (8) in der Messkopfeinheit (6) zumindest ein Schleifkontaktelement (14) oder zumindest ein leitfähiges Lagerelement vorgesehen sind.

12. Verfahren zur Messung der Verteilung der Restfeuchtegehaltes (RF) einer Materialbahn (4) mittels einer Anordnung (1) bestehend aus zumindest einer Messkopfeinheit (6) mit einer ersten Sensoreinheit (8) und einer zweiten gegenüberliegenden Sensoreinheit (3), bei dem die Materialbahn (4) in einer Förderrichtung (FR) zwischen der ersten und zweiten Sensoreinheit (8, 3) bewegt wird, wobei die bewegte Materialbahn (4) mit der ersten und zweiten Sensoreinheit (8, 3) in Kontakt steht, **dadurch gekennzeichnet, dass** die erste als elektrisch leitfähige Messkugel ausgebildete Sensoreinheit (8), welche drehbar in der Messkopfeinheit (6) gelagert ist, quer zur Förderrichtung (FR) über die bewegte Materialbahn (4) geführt wird, dass der elektrische Widerstand der bewegten Materialbahn (4) zwischen der elektrisch leitfähigen Messkugel (8) und der zweiten Sensoreinheit (3) an unterschiedlichen quer zur Förderrichtung (FR) befindlichen Berührungs- bzw. Messpunkten (BP) gemessen wird und dass abhängig davon jeweils die Verteilung des Restfeuchtegehaltes (RF) quer zur Materialbahn bestimmt wird.

13. Verfahren nach Anspruch 12, **dadurch gekennzeichnet, dass** die elektrische leitfähige Messkugel (8) mit einem einstellbaren Anpressdruck (P) auf die Materialbahn (4) gedrückt wird und/oder dass der Anpressdruck (P) über eine Federeinheit oder ein pneumatisches Element erzeugt wird.

14. Verfahren nach einem der Ansprüche 12 oder 13, **dadurch gekennzeichnet, dass** die Messkopfeinheit (6) über eine Traversiervorrichtung (5) quer zur Materialbahn verschoben wird.

15. Verfahren nach einem der Ansprüche 12 bis 14, **dadurch gekennzeichnet, dass** die elektrisch leitfähige Messkugel (8) mit Druckluft (L) beaufschlagt wird.
